# EUROPÄISCHE PATENTANMELDUNG

(11) **EP 4 570 165 A2**
(43) Veröffentlichungstag der Anmeldung: **18.06.2025**
(21) Anmeldenummer: 24215491.2
(22) Anmeldetag: 26.11.2024
(51) Int. Cl.: A61B 3/13, A61B 90/20, G02B 7/02, G02B 21/00, G02B 21/24

(54) **BEOBACHTUNGSVORRICHTUNG UND VERFAHREN ZUR BEOBACHTUNG EINES AUGES**

(30) Priorität: 15.12.2023 DE 102023135378
(71) Anmelder: Oculus Optikgeräte GmbH, 35582 Wetzlar (DE)
(72) Erfinder: SCHEPP, Oliver, 35423 Lich (DE); RISBY, Lars, 5800 Nyborg (DK); ROMPF, Thomas, 35633 Lahnau (DE)
(74) Vertreter: advotec.

(57) **Zusammenfassung**

Die Erfindung betrifft eine Beobachtungsvorrichtung (10) mit einer Positioniereinheit (11) zur Positionierung einer optischen Einheit (12) in einem Strahlengang (13) eines Mikroskops zwischen einem Objektiv des Mikroskops und vor einem zu beobachtenden Auge und ein Verfahren zur Beobachtung eines Auges mit einer derartigen Beobachtungsvorrichtung, wobei die Positioniereinheit eine Anschlussvorrichtung (14), eine Positioniervorrichtung (15), eine Aufnahmevorrichtung (16) und die optische Einheit umfasst, wobei die optische Einheit eine Linse, die zur Beobachtung eines Augenhintergrundes dient, und ein weiteres optisches Element umfasst, wobei die Positioniereinheit eine Schwenkeinrichtung (19) umfasst, mittels der die optische Einheit aus oder in den Strahlengang schwenkbar ist, wobei mittels der Anschlussvorrichtung die Positioniereinheit an das Mikroskop koppelbar ist, wobei mittels der Aufnahmevorrichtung die Linse an der Positioniervorrichtung adaptiert ist, wobei die Aufnahmevorrichtung zumindest überwiegend, vorzugsweise vollständig, aus Kunststoffmaterial ausgebildet ist, wobei die Positioniervorrichtung einen Tubus (24) aufweist, der an der Schwenkeinrichtung schwenkbar angeordnet ist, wobei der Tubus zumindest überwiegend oder vollständig aus Metall ausgebildet ist.

## Beschreibung

Die Erfindung betrifft eine Beobachtungsvorrichtung mit einer Positioniereinheit zur Positionierung einer optischen Einheit in einem Strahlengang eines Mikroskops zwischen einem Objektiv des Mikroskops und vor einem zu beobachtenden Auge, wobei die Positioniereinheit eine Anschlussvorrichtung, eine Positioniervorrichtung, eine Aufnahmevorrichtung und die optische Einheit umfasst, wobei die optische Einheit eine Linse, die zur Beobachtung eines Augenhintergrunds dient, und ein weiteres optisches Element umfasst, wobei die Positioniereinheit eine Schwenkeinrichtung umfasst, mittels der die optische Einheit aus oder in den Strahlengang schwenkbar ist, wobei mittels der Anschlussvorrichtung die Positioniereinheit an das Mikroskop koppelbar ist, wobei mittels der Aufnahmevorrichtung die Linse an der Positioniervorrichtung adaptiert ist. Weiter betrifft die Erfindung ein Verfahren zur Beobachtung eines Auges mit einer derartigen Beobachtungsvorrichtung, wobei die Aufnahmevorrichtung zumindest überwiegend, vorzugsweise vollständig, aus Kunststoffmaterial ausgebildet ist. Weiter betrifft die Erfindung ein Verfahren zur Beobachtung eines Auges mit einer derartigen Beobachtungsvorrichtung.

Mikroskope zur Durchführung von Augenoperationen werden regelmäßig für Operationen in einem vorderen Bereich eines Auges verwendet. Sollen auch in einem hinteren Bereich des Auges derartige Eingriffe vorgenommen werden, ist es notwendig, das Mikroskop mit einer Beobachtungsvorrichtung zu ergänzen, welche eine Fokussierung eben dieses Bereiches des Auges ermöglicht. Derartige Beobachtungsvorrichtungen umfassen zumindest eine Weitwinkellinse bzw. Ophthalmoskopierlinse zur Weitwinkelbetrachtung des betreffenden hinteren Teils des Auges, wobei die Ophthalmoskopierlinse ein Zwischenbild in einem Strahlengang vor einem Objektiv des Mikroskops zur Verfügung stellt, welches mit dem Mikroskop fokussiert werden kann. Zur Fokussierung des Zwischenbildes bedarf es einer Verkürzung einer Länge des Strahlengangs des Mikroskops, die durch die entsprechende Einstelleinrichtung am Mikroskop vorgenommen werden kann. Da während einer Augenoperation jedoch zwischen verschiedenen Betrachtungsweisen, mit und ohne Ophthalmoskopierlinse gewechselt werden muss, ist eine derartige Einstellung des Mikroskops hinderlich, sodass im Strahlengang vor dem Objektiv eine sogenannte Reduzierlinse vorgesehen sein kann, die zur Verkürzung des Strahlengangs des Mikroskops dient und die gemeinsam mit der Ophthalmoskopierlinse verwendet wird. Die beiden Linsen werden als eine optische Einheit von einer Positioniereinheit der Beobachtungsvorrichtung, welche unmittelbar am Mikroskop befestigt ist, gehaltert und können je nach Bedarf im Strahlengang positioniert werden, ohne dass es einer wesentlichen Anpassung des Mikroskops während einer Operation bedarf. Die Positioniereinheit umfasst regelmäßig eine Anschlussvorrichtung, mittels der die Positioniereinheit an das Mikroskop koppelbar ist. Weiter ist die Positioniereinheit so ausgebildet, dass die betreffenden Linsen einfach in den Strahlengang eingeschwenkt bzw. eingeschoben und wieder daraus entfernt werden können.

Eine derartige Beobachtungsvorrichtung ist beispielsweise aus der DE 10 2011 002 940 A1 bekannt. Um hier eine möglichst genaue Anpassung des Zwischenbildes der Ophthalmoskopierlinse an einer Brennweite des Objektives des Mikroskops vornehmen zu können, ist die Ophthalmoskopierlinse längs des Strahlengangs des Mikroskops mit einem Gewindetrieb einstellbar ausgebildet.

Diese Beobachtungsvorrichtung ist so ausgebildet, dass die Ophthalmoskopierlinse relativ zu dem Auge entlang des Strahlengangs verstellt werden kann. Dabei ist es von Vorteil, wenn sich die Ophthalmoskopierlinse möglichst dicht an dem Auge befindet, da dann ein vergleichsweise großer Bereich des Auges gut sichtbar ist. Gleichzeitig muss jedoch auch vermieden werden, dass die Ophthalmoskopierlinse mit dem Auge in Kontakt gelangt. Um eine scharfe Abbildung eines möglichst großen Bereichs des Auges zu erhalten ist es daher stets erforderlich, auch einen Abstand des Mikroskops relativ zu dem Auge zu variieren und mit dem Abstand der Ophthalmoskopierlinse abzustimmen. Wird für einen bestimmten Arbeitsschritt während einer Augenoperation ein anderer Abstand der Ophthalmoskopierlinse zu dem Auge benötigt, beispielsweise, wenn Kammerflüssigkeit des Auges abgesaugt werden soll, ist diese Anpassung der jeweiligen Relativabstände von der Ophthalmoskopierlinse zum Mikroskop und zum Auge sowie das Scharfstellen des dann erhaltenen Bildes erneut vorzunehmen.

Inzwischen wird es als vorteilhaft angesehen auf eine Sterilisation der Beobachtungsvorrichtung bzw. Positioniereinheit verzichten zu können, wenn diese aus Kunststoff ausgebildet ist, und als ein Einwegprodukt benutzt werden kann. So sind Beobachtungsvorrichtungen aus Kunststoff bekannt, die eine einmalige Verwendung der Beobachtungvorrichtung erlauben. Die DE 10 2018 127 469 B4 zeigt eine derartige Beobachtungsvorrichtung.

Nachteilig ist hier jedoch, dass Kunststoff im Gegensatz zu Metall, insbesondere, wenn die Positioniervorrichtung oder die Positioniereinheit aus fragilen Kunststoffstreben ausgebildet ist, nicht immer in der für die zu positionierende Ophthalmoskopierlinse erforderlichen gewünschten Genauigkeit benutzt werden kann. So kann es leicht zu einer Verschiebung der Ophthalmoskopierlinse längs des Strahlengangs oder einem Versatz quer dazu kommen, wenn die Ophthalmoskopierlinse in den Strahlengang ein- oder ausgeschwenkt wird. Dies erfordert dann regelmäßig eine Korrektur der Position der Ophthalmoskopierlinse, was für die Durchführung einer Augenoperation hinderlich ist.

Der vorliegenden Erfindung liegt daher die Aufgabe zugrunde, eine Beobachtungsvorrichtung und ein Verfahren zur Beobachtung eines Auges vorzuschlagen, die bzw. das eine verbesserte Handhabung während einer Augenoperation ermöglicht.

Diese Aufgabe wird durch eine Beobachtungsvorrichtung mit den Merkmalen des Anspruchs 1, ein Mikroskop mit den Merkmalen des Anspruchs 16 und ein Verfahren mit den Merkmalen des Anspruchs 17 gelöst.

Bei der erfindungsgemäßen Beobachtungsvorrichtung mit einer Positioniereinheit zur Positionierung einer optischen Einheit in einem Strahlengang eines Mikroskops zwischen einem Objektiv des Mikroskops und vor einem zu beobachtenden Auge umfasst die Positioniereinheit eine Anschlussvorrichtung, eine Positioniervorrichtung, eine Aufnahmevorrichtung und die optische Einheit, wobei die optische Einheit eine Linse, die zur Beobachtung eines Augenhintergrunds dient, und ein weiteres optisches Element umfasst, wobei die Positioniereinheit eine Schwenkeinrichtung umfasst, mittels der die optische Einheit aus oder in den Strahlengang schwenkbar ist, wobei mittels der Anschlussvorrichtung die Positioniereinheit an das Mikroskop koppelbar ist, wobei mittels der Aufnahmevorrichtung die Linse an der Positioniereinheit adaptiert ist, wobei die Aufnahmevorrichtung, zumindest überwiegend, vorzugsweise vollständig, aus Kunststoffmaterial ausgebildet ist, wobei die Positioniervorrichtung einen Tubus aufweist, der an der Schwenkeinrichtung schwenkbar angeordnet ist, wobei der Tubus zumindest überwiegend oder vollständig aus Metall ausgebildet ist.

Die erfindungsgemäße Beobachtungsvorrichtung kann mittels der Anschlussvorrichtung an ein Mikroskop adaptiert bzw. lösbar mit diesem verbunden werden. Die Linse, welche eine Ophthalmoskopierlinse sein kann, wird mittels der Positioniereinheit dann in den Strahlengang des Objektivs zwischen dem zu beobachtenden Auge und dem Objektiv gehaltert. Dabei ist vorgesehen, die Linse so anzuordnen, dass eine Hauptachse bzw. optische Achse des Objektivs des Mikroskops durch einen Mittelpunkt der Linse verläuft. Mittels der Schwenkeinrichtung kann die optische Einheit mit der Linse und dem weiteren optischen Element, welches vorzugsweise eine Linse mit positiver Brechkraft ist, während einer Augenoperation nach Bedarf in den Strahlengang eingeschwenkt oder auch wieder ausgeschwenkt werden. Hierbei ist es zunächst unerheblich wie die Schwenkeinrichtung ausgebildet ist, wesentlich ist, dass die optische Einheit vollständig aus dem Strahlengang entfernt und in diesen hineinbewegt werden kann. Die Schwenkeinrichtung kann daher auch als eine Verschiebeeinrichtung verstanden werden, mittels der die optische Einheit parallel zum Strahlengang verschiebbar ist.

Weiter ist vorteilhaft, dass die Aufnahmevorrichtung, welche die Linse haltert, im Wesentlichen aus Kunststoffmaterial und der Tubus im Wesentlichen aus Metall ausgebildet ist. Der schwenkbare Tubus kann so besonders stabil und präzise ausgeführt werden und ermöglicht eine genaue Positionierung der Linse und des weiteren optischen Elements in dem Strahlengang, ohne dass es hier erforderlich wäre, eine korrigierende Justierung vornehmen zu müssen, wie dies bei reinen Einwegprodukten der Fall ist. Gleichfalls ist es möglich, die Aufnahmevorrichtung mit der Linse, aufgrund dessen, dass die Aufnahmevorrichtung aus Kunststoff besteht, besonders kostengünstig herzustellen. Die Herstellung kann einfach und in großer Stückzahl, beispielsweise im Rahmen eines Spritzgussverfahrens, erfolgen. Dies ermöglicht es wiederum, die Aufnahmevorrichtung als ein Einwegprodukt zu nutzen. Die Aufnahmevorrichtung kann dann nach der Durchführung einer Augenoperation entsorgt werden. Eine Sterilisation der Aufnahmevorrichtung ist nicht erforderlich. Zur Durchführung einer nachfolgenden Augenoperation kann eine neue Aufnahmevorrichtung, die steril abgepackt sein kann, verwendet werden. Die Aufnahmevorrichtung kann einfach an dem Tubus adaptiert bzw. lösbar mit diesem verbunden sein.

Das weitere optische Element kann unterhalb der Schwenkeinrichtung angeordnet sein. Dadurch, dass die Positioniervorrichtung einen Tubus aufweist, der an der Schwenkeinrichtung schwenkbar angeordnet ist, wird eine Anordnung des weiteren optischen Elements in den Tubus ermöglicht. Eine bewegbare Anordnung des weiteren optischen Elements innerhalb des Tubus ist so besonders einfach möglich. Dabei kann das weitere optische Element auch leicht von äußeren Einflüssen geschützt werden.

Mittels der Positioniervorrichtung kann das weitere optische Element relativ zum Mikroskop in Längsrichtung des Strahlengangs bewegbar sein. Durch die bewegbare Anordnung des weiteren optischen Elements unterhalb der Schwenkeinrichtung wird es dann möglich, mit dem weiteren optischen Element den Strahlengang des Mikroskops anzupassen bzw. diesen Strahlengang soweit zu verkürzen, dass ein Zwischenbild der Linse fokussiert werden kann. Wenn das weitere optische Element unterhalb der Schwenkeinrichtung entlang des Strahlengangs bewegbar ist, steht vergleichsweise mehr Raum zur Bewegung des weiteren optischen Elements entlang des Strahlengangs zur Verfügung, als bei einer Anordnung des weiteren optischen Elements oberhalb der Schwenkeinrichtung. Hier ist ein Abstand von Schwenkeinrichtung und Objektiv des Mikroskops vergleichsweise kurz, da nur so ein vollständiges Entfernen der optischen Einheit bzw. der Positioniervorrichtung aus dem Strahlengang gewährleistet werden kann. Der vergleichsweise größere Verstellbereich des weiteren optischen Elements ermöglicht es die Beobachtungsvorrichtung universell an verschiedene Mikroskoptypen anzupassen und damit für diese zu nutzen. Es ist dann nicht mehr erforderlich das weitere optische Element individuell für jeweils unterschiedliche Mikroskope mit unterschiedlichen Strahlengängen auszubilden. Darüber hinaus kann auch die Linse fest in einer Position in dem Strahlengang verbleiben und muss nicht relativ zu dem Mikroskop entlang des Strahlengangs bewegt werden. Es ist lediglich erforderlich das Mikroskop zusammen mit der Linse auf das Auge auszurichten. Die Anpassung des Strahlengangs kann dann einfach durch die Bewegung des weiteren optischen Elements ausgeführt werden. Die Linse und das weitere optische Element können jeweils aus einer Mehrzahl von optischen Komponenten gebildet sein, die miteinander verbunden sind und jeweils zusammen ein optisches Element ausbilden.

Die Linse kann eine Ophthalmoskopierlinse sein, wobei das weitere optische Element zumindest eine Linse mit positiver Brechkraft sein kann, die zur Anpassung des Strahlengangs dient, wobei die Linse in dem Tubus unterhalb der Schwenkeinrichtung in Längsrichtung des Strahlengangs bewegbar angeordnet sein kann. Die Linse mit positiver Brechkraft kann eine sogenannte Reduzierlinse sein, mittels der der Strahlengang des Mikroskops verkürzt werden kann. Dadurch, dass die Linse mit positiver Brechkraft in dem Tubus unterhalb der Schwenkeinrichtung längs des Strahlengangs bewegbar ist, kann die Anpassung des Strahlengangs mit einem vergleichsweise großen Verstellbereich einfach vorgenommen werden. Das Verschieben der Linse mit positiver Brechkraft entlang des Strahlengangs kann einfach durch einen Gewindetrieb, eine innerhalb des Tubus ausgebildete Wendel oder dergleichen erfolgen. Hierbei kann vorgesehen sein, dass der Tubus zumindest teilweise rotiert wird. Die Linse mit positiver Brechkraft kann dabei in einer Fassung aufgenommen sein, die zusammen mit der Linse innerhalb des Tubus verschiebbar ist.

Die Beobachtungsvorrichtung kann eine Abdeckeinheit aus Kunststoffmaterial zur sterilen Abdeckung des schwenkbaren Tubus der Positioniervorrichtung umfassen. Die Abdeckeinheit kann vergleichsweise dünnwandig ausgebildet sein, sodass die Abdeckeinheit dicht an dem schwenkbaren Tubus anliegen kann. Das Kunststoffmaterial kann ein starres oder ein flexibles Kunststoffmaterial sein. Weiter kann das Kunststoffmaterial undurchsichtig oder optisch teiltransparent sein. Insbesondere kann die Abdeckeinheit so beschaffen sein, dass der schwenkbare Tubus an seiner Außenfläche vollständig von der Abdeckeinheit gegenüber einer Umgebung abgeschirmt ist. Der schwenkbare Tubus kann dann von einem Operateur manuell erfasst und betätigt werden, auch ohne dass es einer nachfolgenden Sterilisation des Tubus bedarf. Es ist dann lediglich erforderlich, die Abdeckeinheit, die aus Kunststoffmaterial kostengünstig hergestellt werden kann, zu entfernen und gegen eine neue, bisher nicht genutzte, sterile Abdeckeinheit zu ersetzen.

Die Abdeckeinheit kann eine obere sterile Abdeckung, zur zumindest teilweisen Abdeckung einer Stirnfläche des Tubus und eine untere sterile Abdeckung zur zumindest teilweisen Abdeckung einer Umfangsfläche des Tubus, aufweisen. Demnach kann die Abdeckeinheit zumindest zweiteilig ausgebildet sein. Die obere sterile Abdeckung kann von oben auf die Stirnfläche des Tubus aufgesetzt werden, wenn der Tubus aus dem Strahlengang herausgeschwenkt ist. Die untere sterile Abdeckung kann ausgehend von unten auf den Tubus aufgesteckt werden. Der Tubus ist dann allseitig von der Abdeckeinheit umgeben. Insbesondere die obere Sterilabdeckung ermöglicht einen Schutz der Stirnfläche des Tubus vor einer Berührung des Operateurs, wenn dieser den Tubus in einer aus dem Strahlengang herausgeschwenkten Position manuell ergreift. Wenn eine obere Sterilabdeckung verwendet wird, ist es auch von Vorteil, wenn zwischen dem Tubus uns der Anschlussvorrichtung ein Spalt ausgebildet ist, innerhalb dem die obere Sterilabdeckung sich befinden kann, wenn der Tubus in den Strahlengang eingeschwenkt ist.

Die Abdeckeinheit kann zumindest mit einem Verbindungselement ausgebildet sein, welches bei einem Trennen der Abdeckeinheit von der Positioniervorrichtung eine Zerstörung des Verbindungselements erfordert. Das Verbindungselement kann in Art eines Rastelements ausgebildet sein, welches einen am Tubus ausgebildeten Vorsprung übergreift oder in einer am Tubus ausgebildete Nut eingreift. Es kann auch eine Mehrzahl von Verbindungselementen vorgesehen sein. Das Verbindungselement kann flexibel ausgebildet oder gelagert sein, sodass sich das Verbindungselement leicht an den Tubus anlegen kann. Wesentlich ist, dass das Verbindungselement so beschaffen ist, dass bei einem Entfernen der Abdeckeinheit von der Positioniervorrichtung bzw. dem Tubus das Verbindungselement bzw. die Abdeckeinheit zerstört wird. Hierdurch kann verhindert werden, dass die Abdeckeinheit versehentlich erneut verwendet wird.

Die Abdeckeinheit kann zumindest einen Aufreißstreifen aufweisen, mittels dem die Abdeckeinheit zumindest teilweise zerstört werden kann. Der Aufreißstreifen kann mit einer Lasche ausgebildet sein, die manuell leicht ergriffen werden kann. Der Aufreißstreifen kann durch eine Schwächungslinie oder zwei parallele Schwächungslinien in der Abdeckeinheit ausgebildet sein. Wenn die Abdeckeinheit beispielsweise mittels einer Rastverbindung an der Positioniervorrichtung bzw. dem Tubus befestigt ist, kann die Rastverbindung durch eine manuelle Betätigung des Aufreißstreifens zerstört werden. Hierdurch wird es ermöglicht die Abdeckeinheit von der Positioniervorrichtung bzw. dem Tubus leicht zu entfernen.

Die Aufnahmevorrichtung kann mit zumindest einem Verbindungselement ausgebildet sein, welches bei einem Trennen der Aufnahmevorrichtung von der Positioniervorrichtung eine Zerstörung des Verbindungselements erfordert. Auch hierdurch kann verhindert werden, dass die Aufnahmevorrichtung nach dem Trennen von der Positioniervorrichtung versehentlich wiederverwertet wird. Das Verbindungselement kann beispielsweise so ausgebildet sein, dass es zu einem Bruch des Verbindungselements kommt, wenn die Aufnahmevorrichtung von der Positioniervorrichtung entfernt wird.

Die Aufnahmevorrichtung kann mit einem weiteren, vorzugsweise konischen Tubus ausgebildet sein. Der weitere Tubus kann unmittelbar an den Tubus angeschlossen und mit diesem fest verbunden sein. Die feste Verbindung kann beispielsweise durch eine Rastverbindung ausgebildet sein. Besonders vorteilhaft ist es, wenn der weitere Tubus in Art eines Konus ausgebildet ist. Der Tubus kann dann an einen Verlauf eines Strahlengangs so angepasst sein, dass an einem unteren Ende des weiteren Tubus ein Durchmesser des weiteren Tubus vergleichsweise klein ist. An dem unteren Ende kann dann die Linse angeordnet sein. Auch ist es vorteilhaft, wenn der weitere Tubus geschlossen ausgebildet ist. Der weitere Tubus kann dann in Art einer konischen Hülse ausgebildet sein.

Der weitere Tubus kann aus einem oberen Abschnitt und aus einem unteren Abschnitt ausgebildet sein, wobei der untere Abschnitt an dem oberen Abschnitt lose oder federbelastet gelagert sein kann, derart, dass der untere Abschnitt in den oberen Abschnitt hineingeschoben werden kann. Die federbelastete Lagerung kann durch eine Druckfeder ausgebildet sein, die in den oberen Abschnitt eingesetzt ist, und gegen deren Federkraft der untere Abschnitt in den oberen Abschnitt hineinbewegt werden kann. Hierdurch kann verhindert werden, dass bei einer eventuellen Kollision der Linse bzw. des unteren Abschnitts mit einem Auge einer zu operierenden Person das Auge in unerwünschter Weise verletzt wird.

An dem unteren Abschnitt kann zumindest ein manuell betätigbarer Vorsprung ausgebildet sein, wobei der Vorsprung einen in den oberen Abschnitt ausgebildeten Längsschlitz durchgreifen und entlang des Längsschlitzes bewegbar sein kann. Ein Operateur kann dann den unteren Abschnitt in Richtung des Strahlengangs manuell bewegen dadurch, dass der Operateur den Vorsprung ergreift und in Richtung des Mikroskops nach oben hin zieht derart, dass der untere Abschnitt in den oberen Abschnitt hineinbewegt wird. Vorteilhaft können dazu auch zwei gegenüberliegende Vorsprünge an dem unteren Abschnitt ausgebildet sein, die in jeweils einander gegenüberliegende Längsschlitze an dem oberen Abschnitt eingreifen. Die Möglichkeit der manuellen Bewegung des unteren Abschnitts in Richtung des Strahlengangs vom Auge weg ist vorteilhaft von einem Operateur nutzbar, wenn die optische Einheit aus dem Strahlengang herausgeschwenkt werden soll. Insbesondere wenn sich die Linse besonders dicht an dem zu operierenden Auge befindet, kann die Linse aus einem Gefahrenbereich für das Auge manuell herausbewegt und in einer unmittelbar darauffolgenden manuellen Bewegung die optische Einheit aus dem Strahlengang herausgeschwenkt werden. Gleiches betrifft eine umgekehrte Bewegung der optischen Einheit in den Strahlengang hinein. Eine entsprechende Bewegung des Mikroskops ist dann nicht mehr erforderlich.

Die Positioniervorrichtung kann eine Antriebeinheit aufweisen, mittels der eine Position des weiteren optischen Elements in Längsrichtung des Strahlengangs eingestellt werden kann. Die Antriebeinheit kann rein manuell oder auch elektrisch betrieben werden. Wesentlich ist, dass mittels der Antriebeinheit das weitere optische Element längs des Strahlengangs verschiebbar und positionierbar ist. Insofern ist es auch vorteilhaft, wenn die Antriebeinheit selbsthemmend ausgebildet ist. Wenn das weitere optische Element innerhalb des Tubus angeordnet ist, kann die Antriebeinheit zumindest teilweise oder vollständig auch an dem Tubus ausgebildet sein.

Die Antriebeinheit kann an der Anschlussvorrichtung oberhalb der Schwenkeinrichtung und/oder unterhalb der Schwenkeinrichtung an dem Tubus angeordnet sein. Demnach kann die Antriebeinheit allein an dem Tubus angeordnet sein oder auch so ausgebildet sein, dass die Antriebeinheit an der Anschlussvorrichtung und dem Tubus angeordnet ist. Je nach Ausführungsform der Antriebeinheit kann es vorteilhaft sein nur einen Teil der Antriebeinheit an dem Tubus auszubilden, sodass ein Operateur nicht durch ausladende Baugruppen der Beobachtungsvorrichtung bei seiner Tätigkeit behindert ist.

Die Beobachtungsvorrichtung kann eine Abschirmeinheit zur Abschirmung eines optischen Weges der Positioniereinheit umfassen, wobei die Abschirmeinheit aus zumindest einem optisch abgeschirmten oder geschlossenen Tubus gebildet sein kann. Die Positioniervorrichtung und die Aufnahmevorrichtung können beispielsweise diesen geschlossenen Tubus ausbilden. Vorteilhaft kann so vermieden werden, dass bei einer Augenoperation verwendete Lichtquellen, Streulicht oder dergleichen in den Strahlengang gelangen und eine Darstellung des Bildes des Auges, welches durch die optische Einheit von einem Operateur beobachtet wird, in unerwünschter Weise beeinflusst. Eventuelle Helligkeitsunterschiede, Spiegelungen oder dergleichen können so vermieden werden.

Die Antriebeinheit kann einen Schrittmotor umfassen, der über einen Riementrieb oder ein Getriebe an eine Kupplung der Antriebeinheit an den Tubus gekoppelt sein kann. Der Schrittmotor kann dann ein Elektromotor sein, mit dem eine definierte Anzahl Umdrehungen so weit ausführt werden kann, bis sich das weitere optische Element an der gewünschten Position des Tubus befindet. Dazu kann der Tubus abschnittsweise drehbar ausgebildet sein, sodass über den Riementrieb und/oder ein Getriebe Umdrehungen des Schrittmotors auf den Tubus übertragen werden können. Die Antriebeinheit kann beispielsweise eine Hülse innerhalb des Tubus umfassen, die mit einem Gewinde oder einer Wendel ausgebildet ist und über die Kupplung mit dem Riementrieb und/oder dem Getriebe verbunden ist. Eine Drehung der Hülse, die durch den Schrittmotor bewegt werden kann, kann dann ein Anheben oder Absenken des weiteren optischen Elements bzw. eine Bewegung desselben entlang des Strahlengangs ausführen.

Die Kupplung kann mittels der Schwenkeinrichtung trennbar oder verbindbar sein. Dies ist insbesondere dann vorteilhaft, wenn der Schrittmotor mit dem Riementrieb oder dem Getriebe oberhalb der Schwenkeinrichtung an der Anschlussvorrichtung angeordnet ist. Die Kupplung kann dann zwischen der Anschlussvorrichtung und dem Tubus so ausgebildet sein, dass bei einem Verschwenken des Tubus aus dem Strahlengang hinaus die Kupplung getrennt wird und bei einem Verschwenken des Tubus in den Strahlengang hinein die Kupplung verbunden wird. Die Kupplung kann als eine kraftschlüssige, formflüssige und/oder reibschlüssige Kupplung ausgebildet sein.

Vorteilhaft ist es, wenn die Kupplung eine Magnetkupplung ist, die aus zwei Ringen gebildet ist, die mittels Magneten ein Drehmoment übertragen können, wobei durch eine Rotation des Tubus das weitere optische Element bewegbar sein kann. Die zwei koaxialen Ringe können jeweils eine Anzahl von Magneten aufweisen, die eine Magnetkraft so aufeinander bewirken, dass die Ringe sich einander anziehen und so das Drehmoment übertragen werden kann. Die Magnete können in einem gleichmäßigen Abstand an einer axialen Stirnseite der jeweiligen Ringe angeordnet sein. Die Pole der Magnete der jeweiligen Ringe können sich abwechseln, sodass sich die Ringe in einer definierten Relativposition befinden, wenn die Kupplung geschlossen ist. Besonders vorteilhaft ist, wenn zwischen den Ringen ein Spalt ausgebildet ist, da dann die Ringe bzw. die Kupplung zur Übertragung des Drehmoments nicht aneinander anliegen müssen. Der Spalt kann dazu genutzt werden, eine Sterilabdeckung in die Kupplung bzw. zwischen den Tubus und der Anschlussvorrichtung einzulegen.

Die Positioniereinheit kann eine Steuervorrichtung umfassen, wobei die Steuervorrichtung ausgebildet sein kann, ein aus oder in den Strahlengang schwenkente optischen Einheit zu erfassen und an das Mikroskop zu übermitteln. Das Erfassen des Aus- oder Einschwenkens der optischen Einheit bzw. des Tubus kann leicht mittels eines Sensors der Steuervorrichtung erfolgen. Die Steuervorrichtung kann dann dem Mikroskop signalisieren, ob die optische Einheit in den Strahlengang eingeschwenkt oder ausgeschwenkt ist. Wenn das Mikroskop mit einem sogenannten Inverter ausgestattet ist, kann das Mikroskop den Inverter in den Strahlengang innerhalb des Mikroskops hineinbewegen oder herausbewegen. Mit dem Inverter kann dann eine Strahlvertauschung und ein Spiegelbild des Zwischenbilds der Linse erzeugt werden, sodass ein Operateur bei in den Strahlengang eingeschwenkter optischer Einheit ein lagerichtiges Bild des Auges dargeboten bekommt.

Die Positioniereinheit kann eine Steuervorrichtung umfassen, wobei mittels eines Sensors der Steuervorrichtung eine Rotation des Tubus detektiert werden kann, wobei die Antriebeinheit mittels der Steuervorrichtung derart gesteuert werden kann, dass mittels der Antriebeinheit das weitere optische Element an eine vorausgesetzte Position in Längsrichtung des Strahlengangs bewegt werden kann. Der Sensor kann beispielsweise ein Hall-Sensor sein, der an der Antriebeinheit bzw. dem Tubus angeordnet ist. An dem Tubus kann eine Marke, eine Anzahl von Marken in Art einer Skala oder dergleichen, angeordnet sein, sodass mittels des Sensors eine Drehung und Position des weiteren optischen Elements in Längsrichtung des Strahlengangs detektiert werden kann. Hierdurch ist es dann möglich, eine Position des weiteren optischen Elements entlang eines Verstellbereichs zu detektieren. Kommt es beispielsweise zu einer unbeabsichtigten Rotation des Tubus oder zu einer Rotation der Anschlussvorrichtung an dem Objektiv des Mikroskops, wenn die optische Einheit bzw. der Tubus aus dem Strahlengang herausgeschwenkt wird, befindet sich das weitere optische Element beim Einschwenken in den Strahlengang nicht mehr in der vorausgesetzten Position bzw. nicht mehr in einem Fokus, der von dem Operateur vor dem Herausschwenken eingestellt wurde. Die Steuervorrichtung kann nun mittels der Antriebeinheit das optische Element an die vorausgesetzte Position bzw. den zuvor eingestellten Fokus der optischen Einheit bewegen. Es ist dann nicht mehr erforderlich, dass der Operateur die Antriebeinheit betätigt um eine veränderte Einstellung des weiteren optischen Elements zu korrigieren.

Das erfindungsgemäße Mikroskop umfasst eine erfindungsgemäße Beobachtungsvorrichtung. Weitere vorteilhafte Ausführungsformen des Mikroskops ergeben sich aus den Merkmalsbeschreibungen der der auf den Anspruch 1 rückbezogenen Unteransprüche.

Bei dem erfindungsgemäßen Verfahren zur Beobachtung eines Auges mit einer Beobachtungsvorrichtung wird mittels einer Positioniereinheit der Beobachtungsvorrichtung eine optische Einheit in einem Strahlengang eines Mikroskops zwischen einem Objektiv des Mikroskops und vor einem zu beobachtenden Auge positioniert, wobei die Positioniereinheit eine Anschlussvorrichtung, eine Positioniervorrichtung, eine Aufnahmevorrichtung und die optische Einheit umfasst, wobei die optische Einheit eine Linse, die zur Beobachtung eines Augenhintergrundes dient, und ein weiteres optisches Element umfasst, wobei die Positioniereinheit eine Schwenkeinrichtung umfasst, mittels der die optische Einheit aus oder in den Strahlengang geschwenkt wird, wobei mittels der Anschlussvorrichtung die Positioniereinheit an das Mikroskop gekoppelt wird, wobei mittels der Aufnahmevorrichtung die Linse an der Positioniereinheit adaptiert wird, wobei die Aufnahmevorrichtung zumindest überwiegend, vorzugsweise vollständig, aus Kunststoffmaterial ausgebildet ist, wobei ein an der Schwenkeinrichtung angeordneter Tubus der Positioniervorrichtung geschwenkt wird, wobei der Tubus zumindest überwiegend oder vollständig aus Metall ausgebildet ist. Zu den Vorteilen des erfindungsgemäßen Verfahrens wird auf die Vorteilsbeschreibung der erfindungsgemäßen Beobachtungsvorrichtung verwiesen.

Das weitere optische Element kann zur Korrektur von refraktiven Fehlern des Auges verwendet werden. Da das weitere optische Element eine Fokussierung eines Zwischenbilds der Linse, und damit eine Anpassung des Strahlengangs des Mikroskops ermöglicht, kann auch ein refraktiver Fehler des Auges mit dem weiteren optischen Element korrigiert werden.

Weitere vorteilhafte Ausführungsformen des Verfahrens ergeben sich aus den Merkmalsbeschreibungen der auf den Anspruch 1 rückbezogenen Unteransprüche.

Nachfolgend wird eine bevorzugte Ausführungsform der Erfindung unter Bezugnahme auf die beigefügten Zeichnungen näher erläutert.

Es zeigen:
- **Fig. 1**: eine perspektivische Ansicht einer Beobachtungsvorrichtung;
- **Fig. 2**: eine Seitenansicht der Beobachtungsvorrichtung mit einer Ab schirmeinheit;
- **Fig. 3**: die Seitenansicht der Beobachtungsvorrichtung ohne die Abschirmeinheit;
- **Fig. 4**: eine Seitenansicht der Beobachtungsvorrichtung mit einer aus einem Strahlengang ausgeschwenkten Positioniervorrichtung;
- **Fig. 5**: eine Längsschnittansicht der Beobachtungsvorrichtung mit der Abschirmeinheit;
- **Fig. 6**: die Längsschnittansicht der Beobachtungsvorrichtung ohne die Abschirmeinheit;
- **Fig. 7**: eine perspektivische Ansicht der Beobachtungsvorrichtung mit der aus dem Strahlengang ausgeschwenkten Positioniervorrichtung und der Abschirmeinheit;
- **Fig. 8**: eine perspektivische Ansicht der Beobachtungsvorrichtung ohne die Abschirmeinheit und eine Aufnahmevorrichtung;
- **Fig. 9**: eine Längsschnittansicht der Abschirmeinheit und der Aufnahmevorrichtung;
- **Fig. 10**: eine Explosionsdarstellung der Abschirmeinheit und der Aufnahmevorrichtung.

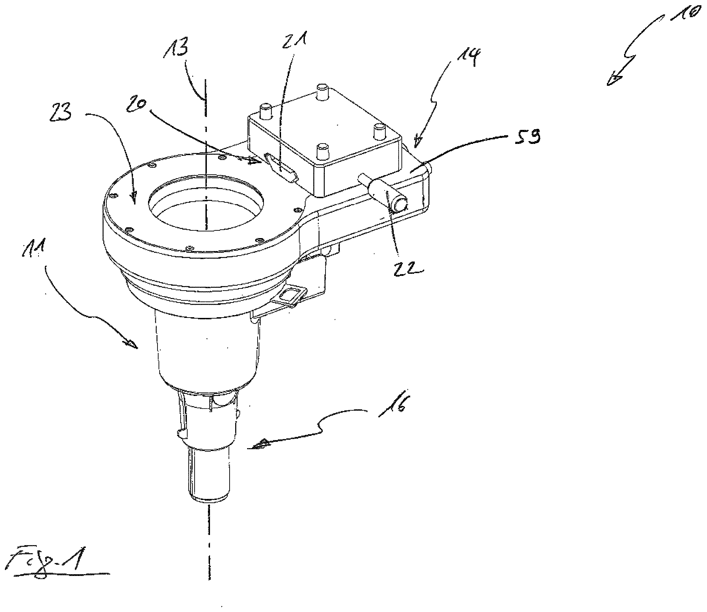

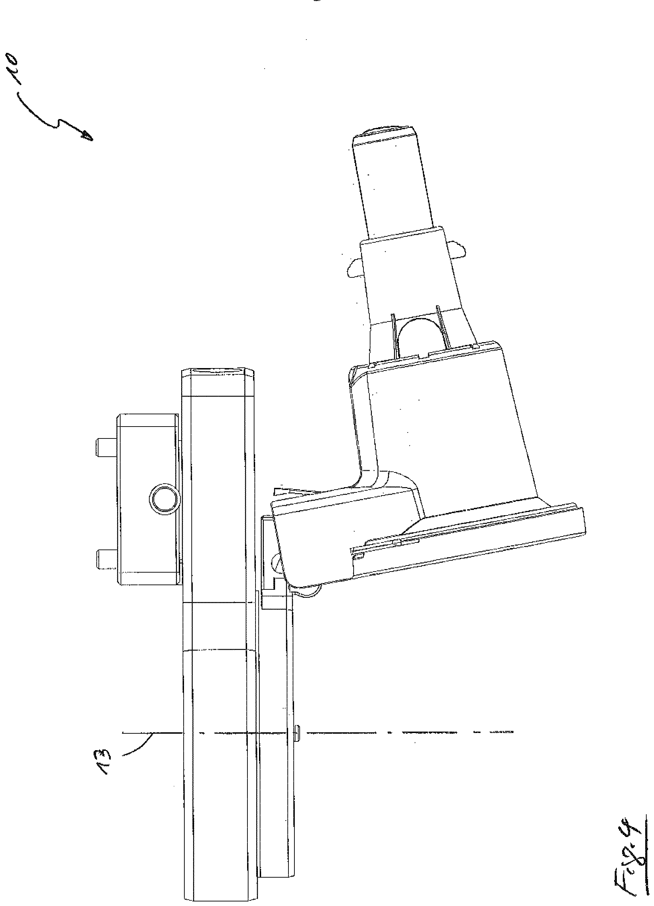

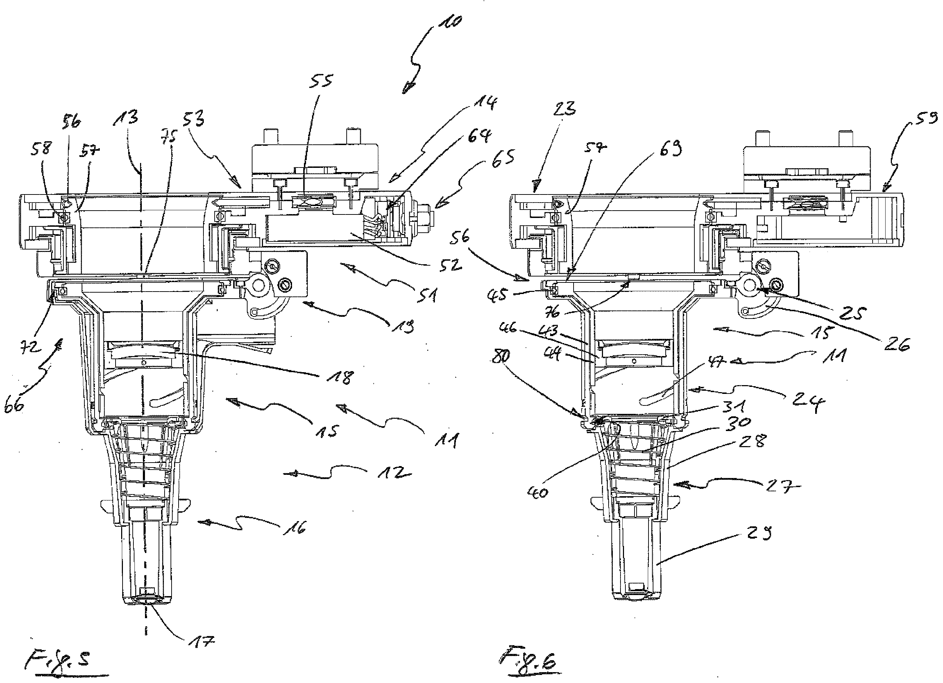

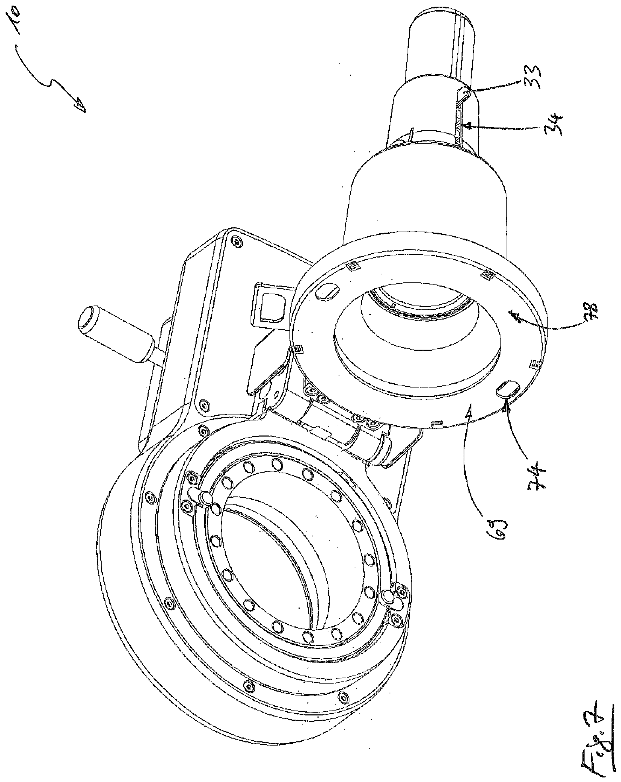

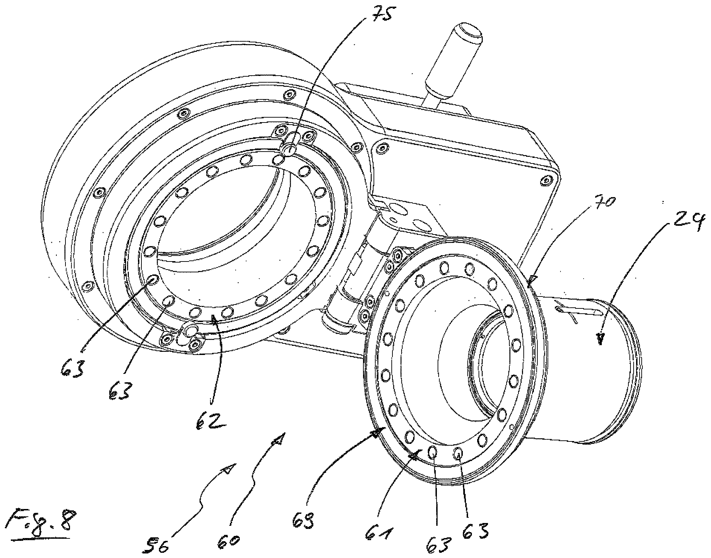

Eine Zusammenschau der **Fig. 1** bis **8** zeigt eine Beobachtungsvorrichtung 10 mit einer Positioniereinheit 11 zur Positionierung einer optischen Einheit 12 in einem Strahlengang 13 eines hier nicht näher dargestellten Mikroskops. Die Beobachtungsvorrichtung 10 ist zwischen einem Objektiv des Mikroskops und vor einem zu beobachtenden Auge an dem Mikroskop adaptierbar. Die Positioniereinheit 11 umfasst eine Anschlussvorrichtung 14, eine Positioniervorrichtung 15, eine Aufnahmevorrichtung 16 sowie die optische Einheit 12. Die optische Einheit 12 ist durch eine Ophthalmoskopierlinse 17 und eine Linse mit positiver Brechkraft bzw. Reduzierlinse 18 gebildet. Die Ophthalmoskopierlinse 17 dient hier zur Beobachtung eines Augenhintergrundes und die Reduzierlinse 18 zur Anpassung des Strahlengangs 13 des Mikroskops an ein hier nicht sichtbares Zwischenbild der Ophthalmoskopierlinse 17. Weiter umfasst die Positioniereinheit 11 eine Schwenkeinrichtung 19 mittels der die optische Einheit 12 in den Strahlengang 13 hinein schwenkbar oder heraus schwenkbar ist. Die **Fig. 1** bis **3****,** **5** und **6** zeigen die in dem Strahlengang 13 eingeschwenkte Positioniervorrichtung 15 mit der optischen Einheit 12 und die **Fig. 4****,** **7** und **8****,** die aus dem Strahlengang 13 ausgeschwenkte Positioniervorrichtung 15 mit der optischen Einheit 12.

Weiter ist die Positioniereinheit 11 mittels der Anschlussvorrichtung 14 an ein Mikroskop koppelbar. Die Anschlussvorrichtung 14 ist hier unter anderem durch eine Aufnahme 20 mit einer Schiene 21 und Spannschraube 22 ausgebildet und an einem Objektiv des Mikroskops so adaptierbar, dass das Objektiv unmittelbar an eine Oberseite 23 der Anschlussvorrichtung 14 angrenzt.

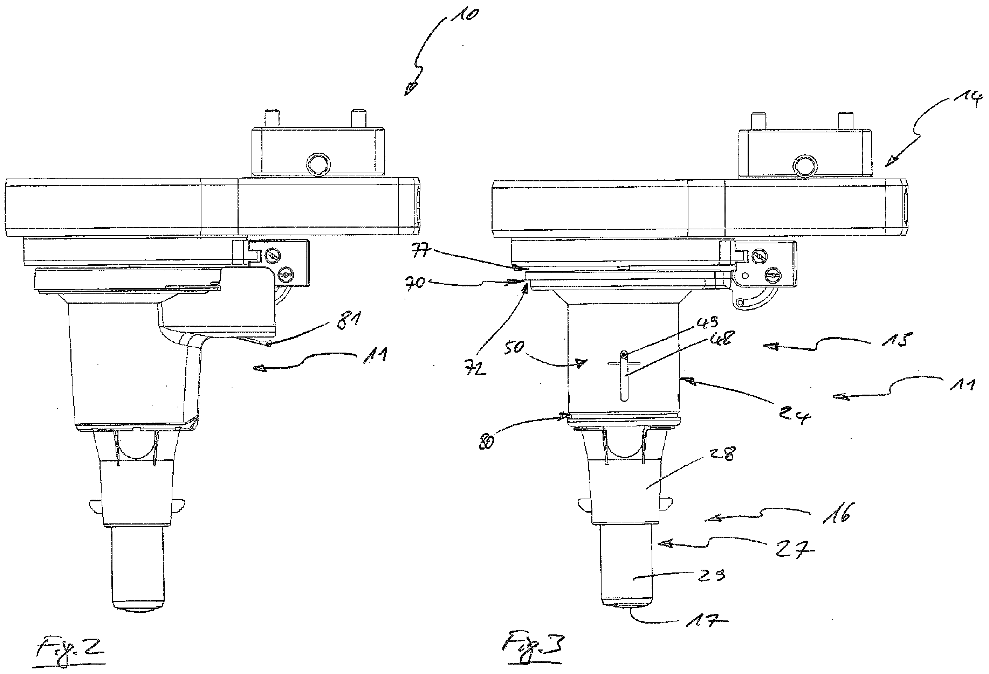

Die Aufnahmevorrichtung 16 ist nahezu vollständig aus Kunststoff ausgebildet und haltert die Ophthalmoskopierlinse 17. Die Aufnahmevorrichtung 16 ist an die Positioniervorrichtung 15 adaptiert. Die Positioniervorrichtung 15 ist im Wesentlichen aus Metall ausgebildet. Ein Tubus 24 der Positioniervorrichtung 15 ist mittels der Schwenkeinrichtung 19, die hier durch ein Scharnier 25 ausgebildet ist, aus einer im Wesentlichen vertikalen Position in dem Strahlengang 13 aus dem Strahlengang 13 heraus über 90 Grad so verschwenkbar, dass der Strahlengang 13 freigegeben ist. Das Scharnier 25 ist mit einer Führung 26 ausgebildet, die ein Verrasten der Positioniervorrichtung 15 in den jeweiligen in den **Fig. 2** und **4** gezeigten Positionen erlaubt. Die Positioniervorrichtung 15 kann so sicher in der jeweiligen Position fixiert werden.

Die Aufnahmevorrichtung 16 ist durch einen weiteren Tubus 27 ausgebildet, der hier aus einem oberen Abschnitt 28 und aus einem unteren Abschnitt 29 gebildet ist. Weiter ist eine Druckfeder 30 in den oberen Abschnitt 28 eingelegt und mittels eines Rings 31 in dem oberen Abschnitt 29 fixiert. Die Ophthalmoskopierlinse 17 ist an einem unteren Ende 32 der Aufnahmevorrichtung 16 gehaltert. Weiter ist der untere Abschnitt 29 mit zwei Vorsprüngen 33 ausgebildet die jeweils einen Längsschlitz 34 in dem oberen Abschnitt 28 durchgreifen. Die Druckfeder 30 liegt an einem oberen Rand 35 des unteren Abschnitts 29 an, wobei der untere Abschnitt 29 mit einer Ringschulter 36 auf einem Absatz 37 an einem unteren Ende 38 des oberen Abschnitts 28 aufliegt. Im Falle einer Kollision des unteren Endes 32 mit einem Auge kann nun der untere Abschnitt 29 in den oberen Abschnitt 28 gegen eine Federkraft der Druckfeder 30 hineingeschoben werden. Weiter ist es auch möglich die Vorsprünge 33 mit einer Hand zu ergreifen und den unteren Abschnitt 29 in den oberen Abschnitt 28 hineinzuschieben, um einen ausreichenden Abstand gegenüber einem Auge herzustellen, wenn die Aufnahmevorrichtung 16 zusammen mit der Positioniervorrichtung 15 verschwenkt werden soll.

Die Aufnahmevorrichtung 16 ist mit Verbindungselementen 39 ausgebildet, die in eine Nut 40 innerhalb des Tubus 40 eingreifen und dort verrastet sind. Die Verbindungselemente 39 sind an Laschen 41 an einem oberen Ende 42 des oberen Abschnitts 28 ausgebildet. Die Laschen 41 erlauben eine federnde Lagerung der Verbindungselemente 39 quer zum Strahlengang 13 und können auch manuell betätigt werden. Ein Zusammendrücken bzw. eine Pronation der Laschen 41 erlaubt dann ein Entfernen der Aufnahmevorrichtung 16 von dem Tubus 24.

Der Tubus 24 ist im Wesentlichen aus einer Außenhülse 43 und einer Innenhülse 44 gebildet, wobei die Innenhülse 44 an einem Lager 45 drehbar innerhalb des Tubus 24 befestigt ist. In die Innenhülse 44 ist eine Fassung 46 mit der Reduzierlinse 18 eingesetzt. Weiter ist in der Innenhülse 44 eine Wendel 47 und in der Außenhülse 43 ein Schlitz 48 ausgebildet. Einander gegenüberliegende Vorsprünge 49 an der Fassung 46 durchgreifen jeweils die Wendeln 47 und die Schlitze 48. Eine Drehung der Innenhülse 44 relativ zu der Außenhülse 43 bewirkt so eine Bewegung der Fassung 46 mit der Reduzierlinse 18 entlang des Strahlengangs 13. Eine Position der Reduzierlinse 18 in dem Tubus 24 ist für einen Benutzer an einer Umfangsfläche 50 des Tubus 24 ersichtlich. Hier sind jeweils die Vorsprünge 49 in den Schlitzen 48 sichtbar.

Bei einer Verwendung der Beobachtungsvorrichtung 10 kann zunächst die Ophthalmoskopierlinse 17 durch eine Justierung einer Höhe des Mikroskops auf das Auge ausgerichtet werden. Nachfolgend kann die Reduzierlinse 18 durch Anpassung ihrer Position in dem Tubus 24 so eingestellt werden, dass ein Zwischenbild der Ophthalmoskopierlinse 17 mit dem Mikroskop scharf fokussiert werden kann. Die Drehung der Innenhülse 44 in der Außenhülse 43 wird durch eine Antriebeinheit 51 der Positioniervorrichtung 15 ausgeführt. Die Antriebeinheit 51 ist hier an der Anschlussvorrichtung 14 angeordnet und umfasst einen Schrittmotor 52, einen Riementrieb 53 und eine Kupplung 54. Ein Antriebsrad 55 des Riementriebs 53 ist hier über einen Riemen 56 mit einer Abtriebshülse 57 innerhalb der Anschlussvorrichtung 14 verbunden. Die Abtriebshülse 57 umgibt den Strahlengang 13 koaxial und ist mittels eines Lagers 58 drehbar in einem Gehäuse 59 der Anschlussvorrichtung 14 gelagert. Die Kupplung 54 ist als eine Magnetkupplung 60 ausgebildet, wobei an einer axialen Stirnseite 61 der Innenhülse 44 und in einer gegenüberliegenden axialen Stirnseite 62 der Abtriebshülse 57 jeweils Magnete 63 eingelassen sind. Die Magnete 63 sind mit wechselnder Polung derart angeordnet, dass gegenüberliegende Magnete 63 aufeinander eine Magnetkraft bewirken, sodass bei einer Rotation der Abtriebshülse 57 ein Drehmoment auf die Innenhülse 44 übertragen werden kann.

Innerhalb des Gehäuses 59 befindet sich eine Steuervorrichtung 64 der Positioniereinheit 11 mittels der eine Rotation des Tubus 24 bzw. der Innenhülse 44 gesteuert und detektiert werden kann. Auch bei einer Verdrehung bzw. Rotation der Beobachtungsvorrichtung 10 an dem Mikroskop um den Strahlengang 13, beispielsweise durch eine manuell ausgeführte Drehung, ist es so möglich mittels des Schrittmotors 52 die Reduzierlinse 18 in eine vorausgesetzte Position in Längsrichtung des Strahlengangs 13 zu bringen, wenn durch diese Drehung die Reduzierlinse 18 verstellt bzw. entlang des Strahlengangs 13 bewegt wurde. Hierzu kann die Steuervorrichtung 64 mit einem hier nicht dargestellten Sensor zur Detektion der Drehung ausgestattet sein. Weiter sind an der Steuervorrichtung 64 Anschlüsse 65 zur Verbindung mit einer Stromversorgung, einem hier nicht dargestellten Fußschalter und dem Mikroskop vorgesehen.

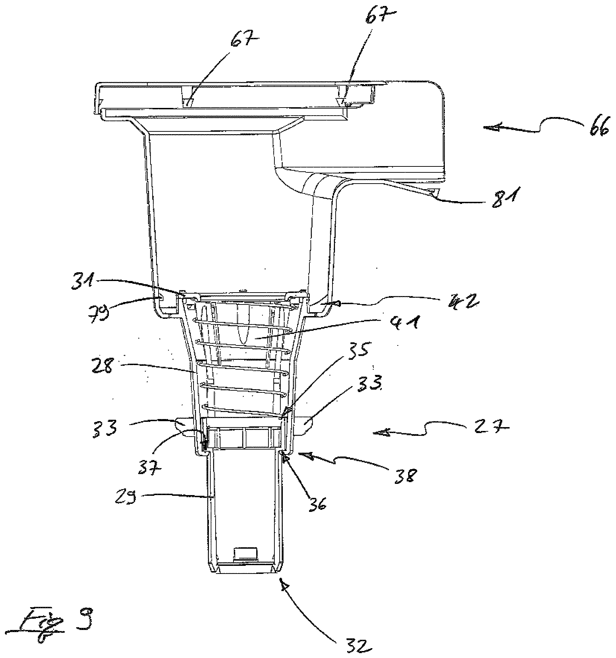

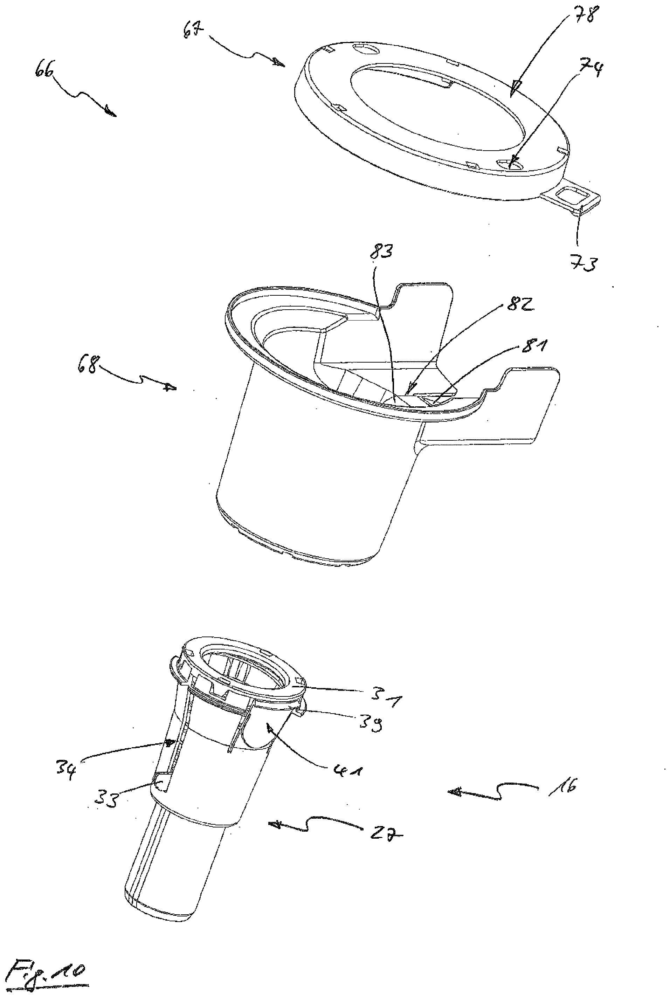

Die **Fig. 9** und **10** zeigen die Aufnahmevorrichtung 16 zusammen mit einer Abdeckeinheit 66 der Beobachtungsvorrichtung 10. Die Abdeckeinheit 66 besteht aus einem Kunststoffmaterial und ist aus einer oberen Sterilabdeckung 67 und einer unteren Sterilabdeckung 68 gebildet. Mit der oberen Sterilabdeckung 67 ist eine Stirnfläche 69 und eine obere Umfangsfläche 70 des Tubus 24 abdeckbar. Mit der unteren Sterilabdeckung 68 ist die Umfangsfläche 50 des Tubus 24 und teilweise das Scharnier 25 abdeckbar. Die obere Sterilabdeckung 67 weist Vorsprünge 71 auf, die eine an dem Tubus 24 ausgebildete obere Ringschulter 72 übergreifen. Die obere Sterilabdeckung 67 kann so an der oberen Ringschulter 72 verrasten. An der oberen Sterilabdeckung 67 ist weiter eine Lasche 73 zum manuellen Entfernen der oberen Sterilabdeckung 67 vorgesehen. Weiter sind Ausnehmungen 74 in der oberen Sterilabdeckung 67 ausgebildet, die in einer Montageposition von Stiften 75 an der Anschlussvorrichtung 14 durchgriffen werden. Die Stifte 75 bilden einen Anschlag 76 für den Tubus 24 und einen Spalt 77 zwischen dem Tubus 24 und der Anschlussvorrichtung 14 aus innerhalb dem ein kreisringförmiger Bedeckungsbereich 78 der oberen Sterilabdeckung 67 aufgenommen und mittels der Stifte 75 gegen Verdrehen gesichert ist.

Die untere Sterilabdeckung 68 ist, wie die obere Sterilabdeckung 67, einstückig ausgebildet und weist Vorsprünge 79 auf, die in eine untere Ringnut 80 des Tubus 24 eingreifen. Die untere Sterilabdeckung 68 kann so einen Tubus 24 durch Verrasten befestigt werden. Weiter ist an der unteren Sterilabdeckung 68 eine Lasche 81 ausgebildet, mittels der die untere Sterilabdeckung 68 von dem Tubus 24 entfernt werden kann. Die Lasche 81 ist mit Schwächungslinien 82 in der unteren Sterilabdeckung 68 ausgebildet, so dass ein Aufreißstreifen 83 ausgebildet wird, der bei einer manuellen Betätigung der Lasche 81 eine Zerstörung der unteren Sterilabdeckung 68 zur Folge hat. So kann sichergestellt werden, dass die Abdeckeinheit 66 nach einem Entfernen nicht erneut wiederverwendet wird. Weiter ist das Kunststoffmaterial der Abdeckeinheit 66 teiltransparent.

Da die Abdeckeinheit 66 den Tubus 24 vollständig abdeckt, ist eine Sterilisation des Tubus 24 nach der Durchführung einer Operation nicht erforderlich. Die Abdeckeinheit 66 kann nach der Operation entfernt und durch eine neue, bisher nicht genutzte sterile Abdeckeinheit 66 ersetzt werden. Dies trifft ebenfalls auf die Aufnahmevorrichtung 16 mit der Ophthalmoskopierlinse 17 zu, sodass auch hier eine ungewollte Wiederverwertung und Sterilisation ausgeschlossen ist. Die Beobachtungsvorrichtung 10 kann so nach der Durchführung einer Operation durch das Auswechseln der Abdeckeinheit 66 und der Aufnahmevorrichtung 16 schnell für eine nachfolgende Operation ertüchtigt werden, ohne dass eine zeitaufwendige Sterilisation der Beobachtungsvorrichtung 10 erforderlich wäre.

## Patentansprüche

1. Beobachtungsvorrichtung (10) mit einer Positioniereinheit (11) zur Positionierung einer optischen Einheit (12) in einem Strahlengang (13) eines Mikroskops zwischen einem Objektiv des Mikroskops und vor einem zu beobachtenden Auge, wobei die Positioniereinheit eine Anschlussvorrichtung (14), eine Positioniervorrichtung (15), eine Aufnahmevorrichtung (16) und die optische Einheit umfasst, wobei die optische Einheit eine Linse, die zur Beobachtung eines Augenhintergrundes dient, und ein weiteres optisches Element umfasst, wobei die Positioniereinheit eine Schwenkeinrichtung (19) umfasst, mittels der die optische Einheit aus oder in den Strahlengang schwenkbar ist, wobei mittels der Anschlussvorrichtung die Positioniereinheit an das Mikroskop koppelbar ist, wobei mittels der Aufnahmevorrichtung die Linse an der Positioniervorrichtung adaptiert ist, wobei die Aufnahmevorrichtung zumindest überwiegend, vorzugsweise vollständig, aus Kunststoffmaterial ausgebildet ist,
**dadurch gekennzeichnet,**
**dass** die Positioniervorrichtung einen Tubus (24) aufweist, der an der Schwenkeinrichtung schwenkbar angeordnet ist, wobei der Tubus zumindest überwiegend oder vollständig aus Metall ausgebildet ist.

2. Beobachtungsvorrichtung nach Anspruch 1,
**dadurch gekennzeichnet,**
**dass** das weitere optische Element unterhalb der Schwenkeinrichtung (19) angeordnet ist.

3. Beobachtungsvorrichtung nach Anspruch 1 oder 2,
**dadurch gekennzeichnet,**
**dass** mittels der Positioniervorrichtung (15) das weitere optische Element relativ zum Mikroskop in Längsrichtung des Strahlengangs (13) bewegbar ist.

4. Beobachtungsvorrichtung nach einem der vorangehenden Ansprüche, **dadurch gekennzeichnet,**
**dass** die Linse eine Ophthalmoskopierlinse (17) ist, wobei das weitere optische Element zumindest eine Linse (18) mit positiver Brechkraft ist, die zur Anpassung des Strahlengangs (13) dient, wobei die Linse in dem Tubus (24) unterhalb der Schwenkeinrichtung (19) in Längsrichtung des Strahlengangs bewegbar angeordnet ist.

5. Beobachtungsvorrichtung nach einem der vorangehenden Ansprüche, **dadurch gekennzeichnet,**
**dass** die Beobachtungsvorrichtung (10) eine Abdeckeinheit (66) aus Kunststoffmaterial zur sterilen Abdeckung des schwenkbaren Tubus (24) der Positioniervorrichtung (15) umfasst.

6. Beobachtungsvorrichtung nach Anspruch 5,
dadurch g**ekennzeichnet**,
dass die Abdeckeinheit (66) eine obere Sterilabdeckung (67), zur zumindest teilweisen Abdeckung einer Stirnfläche (69) des Tubus (24) und eine untere Sterilabdeckung (68), zur zumindest teilweisen Abdeckung einer Umfangsfläche (70) des Tubus, aufweist.

7. Beobachtungsvorrichtung nach Anspruch 5 oder 6,
**dadurch gekennzeichnet,**
**dass** die Abdeckeinheit (66) mit zumindest einem Verbindungselement (71, 79) ausgebildet ist, welches bei einem Trennen der Abdeckeinheit von der Positioniervorrichtung (15) eine Zerstörung des Verbindungselements erfordert.

8. Beobachtungsvorrichtung nach einem der Ansprüche 5 bis 7,
**dadurch gekennzeichnet,**
**dass** die Abdeckeinheit (66) zumindest einen Aufreißstreifen (83) aufweist, mittels dem die Abdeckeinheit zumindest teilweise zerstörbar ist.

9. Beobachtungsvorrichtung nach einem der vorangehenden Ansprüche, **dadurch gekennzeichnet,**
**dass** die Aufnahmevorrichtung (16) mit zumindest einem Verbindungselement (39) ausgebildet ist, welches bei einem Trennen der Aufnahmevorrichtung von der Positioniervorrichtung (15) eine Zerstörung des Verbindungselements erfordert.

10. Beobachtungsvorrichtung nach einem der vorangehenden Ansprüche, **dadurch gekennzeichnet,**
**dass** die Aufnahmevorrichtung (16) mit einem weiteren, vorzugsweise konischen Tubus (27) ausgebildet ist.

11. Beobachtungsvorrichtung nach Anspruch 10,
**dadurch gekennzeichnet,**
**dass** der weitere Tubus (27) aus einem oberen Abschnitt (28) und aus einem unteren Abschnitt (29) ausgebildet ist, wobei der untere Abschnitt an dem oberen Abschnitt lose oder federbelastet gelagert ist, derart, dass der untere Abschnitt in den oberen Abschnitt einschiebbar ist.

12. Beobachtungsvorrichtung nach Anspruch 11,
**dadurch gekennzeichnet,**
**dass** an dem unteren Abschnitt (29) zumindest ein manuell betätigbarer Vorsprung (33) ausgebildet ist, wobei der Vorsprung einen in dem oberen Abschnitt (28) ausgebildeten Längsschlitz (34) durchgreift und entlang des Längsschlitzes bewegbar ist.

13. Beobachtungsvorrichtung nach einem der vorangehenden Ansprüche, **dadurch gekennzeichnet,**
**dass** die Positioniervorrichtung (15) eine Antriebeinheit (51) aufweist, mittels der eine Position des weiteren optischen Elements in Längsrichtung des Strahlengangs (13) einstellbar ist.

14. Beobachtungsvorrichtung nach Anspruch 13,
**dadurch gekennzeichnet,**
**dass** die Antriebeinheit (51) an der Anschlussvorrichtung (14) oberhalb der Schwenkeinrichtung (19) und/oder unterhalb der Schwenkeinrichtung an dem Tubus (24) angeordnet ist.

15. Beobachtungsvorrichtung nach einem der vorangehenden Ansprüche, **dadurch gekennzeichnet,**
**dass** die Beobachtungsvorrichtung (10) eine Abschirmeinheit zur Abschirmung eines optischen Weges der Positioniereinheit (11) umfasst, wobei die Abschirmeinheit aus zumindest einem optisch abgeschirmten oder geschlossenen Tubus (24, 27) gebildet ist.

16. Mikroskop mit einer Beobachtungsvorrichtung (10) nach einem der vorangehenden Ansprüche.

17. Verfahren zur Beobachtung eines Auges mit einer Beobachtungsvorrichtung (10), wobei mittels einer Positioniereinheit (11) der Beobachtungsvorrichtung eine optische Einheit (12) in einem Strahlengang (13) eines Mikroskops zwischen einem Objektiv des Mikroskops und vor einem zu beobachtenden Auge positioniert wird, wobei die Positioniereinheit eine Anschlussvorrichtung (14), eine Positioniervorrichtung (15), eine Aufnahmevorrichtung (16) und die optische Einheit umfasst, wobei die optische Einheit eine Linse, die zur Beobachtung eines Augenhintergrundes dient, und ein weiteres optisches Element umfasst, wobei die Positioniereinheit eine Schwenkeinrichtung (19) umfasst, mittels der die optische Einheit aus oder in den Strahlengang geschwenkt wird, wobei mittels der Anschlussvorrichtung die Positioniereinheit an das Mikroskop gekoppelt wird, wobei mittels der Aufnahmevorrichtung die Linse an der Positioniervorrichtung adaptiert wird, wobei die Aufnahmevorrichtung zumindest überwiegend, vorzugsweise vollständig, aus Kunststoffmaterial ausgebildet ist,
**dadurch gekennzeichnet,**
**dass** ein an der Schwenkeinrichtung angeordneter Tubus (24) der Positioniervorrichtung geschwenkt wird, wobei der Tubus zumindest überwiegend oder vollständig aus Metall ausgebildet ist.
